# FASCICULE DE BREVET EUROPEEN

(11) **EP 0 498 716 B1**
(45) Date de publication et mention de la délivrance du brevet: **08.02.1995**
(21) Numéro de dépôt: 92400286.8
(22) Date de dépôt: 05.02.1992
(51) Int. Cl.: A61K 7/50, A61K 7/08, A61K 7/06, A61K 7/48

(54) **Composition de lavage**
Reinigungsmittel
Cleansing composition

(30) Priorité: 06.02.1991 FR 9101330
(43) Date de publication de la demande: 12.08.1992
(73) Titulaire: L'OREAL, 75008 Paris (FR)
(72) Inventeur: Dubief, Claude, F-78150 Le Chesnay (FR); Cauwet, Danièle, F-75011 Paris (FR)
(74) Mandataire: Casalonga, Axel

(56) Documents cités:
- DE-A- 2 851 832
- GB-A- 2 059 959

## Description

L'invention est relative à des compositions de lavage et de conditionnement des matières kératiniques, en particulier les cheveux et/ou de la peau à base d'huile, d'agents tensio-actifs détergents et d'un alcool gras à groupement éther et/ou thioéther ou sulfoxyde, ainsi qu'aux procédés de lavage mettant en oeuvre ces compositions.

Les compositions de lavage des matières kératiniques, notamment les shampooings, sont bien connues dans l'état de la technique. On a déjà proposé dans le passé d'utiliser des huiles dans de telles compositions, notamment dans le but de traiter simultanément au cours du lavage, les cheveux secs afin de leur conférer des propriétés de douceur et de brillance.

Les huiles utilisables dans les compositions de lavage et de conditionnement des matières kératiniques, sont cependant généralement insolubles, de sorte que l'on cherche à maintenir les huiles en dispersion fine et régulière dans le milieu sans cependant faire chuter la viscosité et les propriétés détergentes et moussantes des compositions.

Les huiles doivent également être véhiculées sur les matières kératiniques traitées en vue de leur conférer, suivant l'application, des propriétés de douceur, de brillance et de démêlage.

La demanderesse a découvert, ce qui fait l'objet de l'invention, qu'en utilisant dans des compositions aqueuses de lavage à base d'huiles hydrocarbonées insolubles et d'agents tensio-actifs détergents, au moins un alcool ayant 27 à 44 atomes de carbone et comportant un ou deux groupement(s) éther et/ou thioéther ou sulfoxyde, il était possible de préparer des compositions contenant une huile, présentant une très bonne homogénéité et une stabilité améliorée, ainsi qu'une viscosité satisfaisante pour l'application sur les matières kératiniques, en particulier les cheveux ou la peau.

Les compositions ainsi préparées possèdent également de bonnes propriétés détergentes et moussantes et confèrent aux matières kératiniques, notamment aux cheveux et/ou à la peau, une grande douceur.

Ces compositions, lorsqu'elles sont appliquées sur les cheveux, possèdent, en plus de leurs propriétés lavantes, des propriétés de conditionnement des cheveux. On appelle conditionnement, des propriétés de brillance, de démêlage faciles et de douceur au toucher. On constate, par ailleurs, que la chevelure n'est pas alourdie après plusieurs applications.

L'invention a donc pour objet de nouvelles compositions aqueuses de lavage à base d'huiles hydrocarbonées, d'agents tensio-actifs détergents et d'alcools éther et/ou thioéther ou sulfoxyde, sous forme de suspension.

Un autre objet de l'invention est constitué par le procédé de lavage mettant en oeuvre de telles compositions.

L'invention a également pour objet l'utilisation d'alcools éthers et/ou thioéther ou sulfoxyde définis ci-après comme agents de mise en suspension d'huile dans un milieu aqueux contenant des agents tensio-actifs détergents.

D'autres objets de l'invention apparaîtront à la lecture de la description et des exemples qui suivent.

Les compositions de lavage des matières kératiniques , en particulier des cheveux et de la peau, conformes à l'invention, comprennent dans un milieu aqueux, au moins une huile hydrocarbonée, un agent tensio-actif possédant des propriétés détergentes et au moins un alcool ayant 27 à 44 atomes de carbone et comportant un ou deux groupement(s) éther et/ou thioéther ou sulfoxyde répondant à la formule (I) :

R₁ - X -[C₂H₃(OH)]- CH₂ - Y - R₂ (I)

dans laquelle R₁ et R₂ désignent, indépendamment l'un de l'autre, des groupements alkyle linéaires en C₁₂ à C₂₀;
X désigne un atome d'oxygène, un atome de soufre, un groupement sulfoxyde;
Y désigne un atome d'oxygène, de soufre, un groupement sulfoxyde ou méthylène;
dans le cas où Y désigne un groupement méthylène, la somme du nombre d'atomes de carbone présents dans les groupements R₁ et R₂ a une valeur variant de 24 à 40 et de préférence de 26 à 36, inclus;
lorsque Y ne désigne pas un groupement méthylène, la somme des atomes de carbone de R₁ et R₂ a une valeur variant de 24 à 40 inclus et de préférence de 28 à 36 inclus;
lorsque X ou Y désigne sulfoxyde, Y ou X ne désigne pas soufre.

Les composés utilisés préférentiellement conformément à l'invention, sont ceux dans lesquels X désigne oxygène, Y désigne méthylène, et R₁ et R₂ désignent des radicaux ayant 12 à 18 atomes de carbone.

Les composés de formule (I) sont obtenus par réaction d'un composé à hydrogène actif de formule (II) :

R₁XH (II)

avec un composé comportant un groupement oxirane terminal et répondant à la formule (III) : 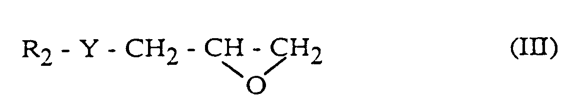
Lorsque X désigne oxygène, ces réactions sont réalisées de préférence en présence d'un excès molaire du composé de formule (II) par rapport au composé de formule (III), cet excès pouvant atteindre cinq fois la quantité stoechiométrique, en présence d'un catalyseur acide tel que par exemple le trifluorure de bore, SnCl₄ ou ZnCl₂ ou en présence d'un catalyseur alcalin tel que le sodium, le potassium, le méthylate, l'éthylate ou le tertiobutylate de sodium ou de potassium.

En catalyse acide, la température de réaction est comprise entre 40°C et 100°C; elle est comprise de préférence en catalyse alcaline entre 80°C et 180°C.

Lorsque l'un des groupements X ou Y désigne un atome de soufre, la réaction est réalisée de préférence en catalyse alcaline et avec des proportions stoechiométriques des composés de formules (II) et (III).

La proportion de catalyseur utilisée est généralement de 0,1 à 3% en poids par rapport au poids de la masse réactionnelle.

Selon le sens d'ouverture de l'époxyde de formule (III), on peut obtenir les isomères de formules (Ia) et (Ib) suivantes : 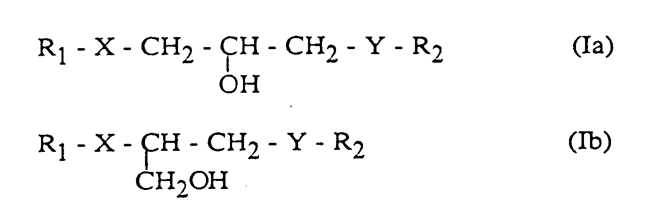
Les composés de formule (I) peuvent être purifiés, après élimination, sous pression réduite, du composé à hydrogène actif de formule (II) résiduel, par distillation moléculaire, de préférence sous environ 10⁻³ mm de mercure (0,13 Pascal).

Lorsque le composé de départ de formule (II) est un alcool, il est également possible d'utiliser, dans le cadre de l'invention, le produit de réaction après simplement élimination partielle ou totale de l'excès d'alcool de formule (II), voire le produit brut de réaction, c'est-à-dire en conservant l'excès d'alcool gras de formule (II) dans sa totalité. En effet, les alcools gras de formule (II) utilisés dans le cadre de la préparation des composés de formule (I) utilisés conformément à l'invention, ne sont pas préjudiciables aux propriétés attendues des composés de formule (I) et ils peuvent contribuer à la stabilisation et à l'opacification des compositions détergentes.

Les composés de formule (I) dans laquelle au moins un des groupements X ou Y désigne un atome de soufre, peuvent être oxydés, selon des procédés classiques, en sulfoxydes qui répondent à la formule (IV) :

R₁ - X₁ -[C₂H₃(OH)]- CH₂ - Y₁ - R₂ (IV)

dans laquelle R₁ et R₂ ont la même signification que dans la formule (I); X₁ désigne un atome d'oxygène ou un groupement sulfoxyde; Y₁ désigne un atome d'oxygène, un groupement méthylène ou sulfoxyde, au mois un des symboles X₁ ou Y₁ représente un groupement sulfoxyde.

On appelle "huile hydrocarbonée", un composé liquide à température ambiante insoluble dans l'eau et à caractère hydrophobe.

Les huiles hydrocarbonées utilisées conformément à l'invention, sont des huiles de synthèse, des huiles minérales, végétales ou animales, les alcools gras insaturés, des esters d'acides gras et de mono- ou polyalcools inférieurs en C₂-C₄.

Parmi les huiles de synthèse, on peut citer les isoparaffines, répondant à la formule : 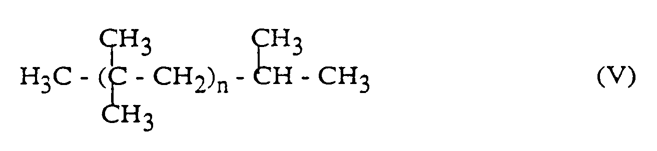
dans laquelle n varie de 2 à 16.

Ces isoparaffines peuvent être utilisées seules ou en mélange avec d'autres isoparaffines de poids moléculaire plus élevé, et répondant à la formule (VI) : 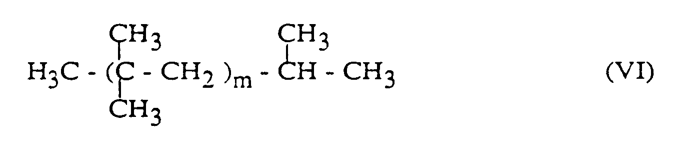
où m est supérieur ou égal à 18 et compris de préférence entre 18 et 40.

Parmi les composés de formule (V), on peut citer plus particulièrement les produits vendus sous la dénomination PERMETHYL 99A, 101A, 102A ou 104A, correspondant respectivement aux valeurs de n 2, 3, 4 et 16, vendus par la Société PRESPERSE INC. ou les produits vendus sous la dénomination ARLAMOL HD par la Société ICI, correspondant à la formule (V), dans laquelle n est égal à 3.

Parmi les composés de formule (VI), on peut citer le produit vendu sous la dénomination PERMETHYL 106A, dans lequel m est égal à 38.

Une autre classe d'huiles de synthèse est constituée par les triglycérides d'acides gras en C₆-C₁₂.

Parmi les huiles minérales, on peut citer l'huile de vaseline.

Les huiles animales sont choisies parmi les huiles naturellement ou chimiquement saturées telles que le squalane, mais on peut citer aussi l'huile de baleine, de phoque, de menhaden, de foie de flétan, de foie de morue, de thon, de suif, de boeuf, de cheval, de mouton, de vison, de loutre.

Parmi les huiles végétales, on peut citer les huiles d'amande, d'arachide, de germe de blé, de lin, de noyaux d'abricots, de noix, de palme, de pistache, de sésame, d'oeillette, de pin, de ricin, de soja, d'avocat, de carthame, de coco, de noisette, d'olive, de pépins de raisins, de tournesol, de colza, de cade, de germe de maïs, de noyaux de pêches, de café, de jojoba, etc. Les huiles naturellement ou chimiquement saturées sont préférées.

Les huiles précitées peuvent être utilisées en mélange dans les compositions conformes à l'invention.

Les agents tensio-actifs utilisés dans les compositions de lavage conformes à l'invention, sont choisis parmi les agents tensio-actifs anioniques, amphotères, zwittérioniques, non-ioniques ou leurs mélanges ayant des propriétés détergentes.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates.

Le radical alkyle ou acyle de ces différents composés est généralement constitué par une chaîne carbonée comportant de 12 à 20 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 20 atomes de carbone.

On peut également utiliser des agents tensio-actifs considérés comme faiblement anioniques tels que les acides éthers carboxyliques polyoxyalkylénés.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acides gras de sucrose, les esters d'acides gras du polyéthylèneglycol, les alkyl (C₈-C₁₈)polyglucosides, les oxydes d'amines tels que les oxydes d'alkylamines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2.528.378 et 2.781.354 et classés dans le dictionnaire CTFA, 3ème édition, 1982, sous la dénomination d'Amphocarboxyglycinates et Amphocarboxypropionates.

Les huiles hydrocarbonées sont utilisées dans les compositions conformes à l'invention dans des proportions comprises de préférence entre 0,05 et 20% et de préférence entre 0,1 et 10% en poids par rapport au poids total de la composition.

Les alcools à groupement(s) éther et/ou thioéther ou sulfoxyde répondant à la formule (I) ou à la formule (IV) utilisés conformément à l'invention sont présents dans des proportions suffisantes pour assurer la mise en suspension des huiles hydrocarbonées dans les compositions et de préférence dans des proportions comprises entre 0,1 et 20% en poids par rapport au poids total de la composition et en particulier entre 0,5 et 10%.

Les agents tensio-actifs sont utilisés dans les compositions conformes à l'invention dans des proportions suffisantes pour conférer un caractère détergent à la composition et sont comprises de préférence entre 5 et 50% en poids par rapport au poids total de la composition et en particulier entre 8 et 35%.

Les compositions, selon l'invention, présentent un pH généralement compris entre 2 et 9, et plus particulièrement entre 3 et 8.

Le milieu aqueux des compositions est constitué, soit par de l'eau, soit par un mélange d'eau et de solvant(s) choisi(s) parmi les alcools inférieurs, les alkylèneglycols et les éthers de glycol; l'eau étant présente dans des proportions supérieures à 20% et de préférence supérieures à 45%.

Les compositions, selon l'invention, peuvent contenir également des agents régulateurs de viscosité, tels que des électrolytes comme le chlorure de sodium ou le xylènesulfonate de sodium, des hydrotropes, des épaississants comme les dérivés de la cellulose, tels que par exemple la carboxyméthylcellulose, l'hydroxypropylcellulose, l'hydroxyéthylcellulose, la gomme de guar, des gommes de guar hydroxypropylées, les scléroglucanes, la gomme de xanthane.

Ces agents régulateurs de viscosité sont utilisés dans des proportions allant jusqu'à 15% en poids par rapport au poids total de la composition et de préférence inférieure à 6%.

Les compositions conformes à l'invention peuvent éventuellement contenir en outre d'autres agents, pourvu qu'ils n'altèrent pas la stabilité des compositions, tels que des tensio-actifs cationiques, des polymères ou des protéines quaternisées ou non, ou des huiles, cires, gommes ou résines de silicone.

Les polymères, les tensio-actifs cationiques et les protéines quaternisées ou non, les silicones, sont utilisés dans les compositions cosmétiques ou dermatologiques, selon l'invention, dans des proportions comprises entre 0,05 et 6% et de préférence entre 0,1 et 3% par rapport au poids total de la composition.

Les compositions selon l'invention peuvent également contenir différents adjuvants habituellement utilisés en cosmétique, tels que des parfums, des conservateurs, des séquestrants, des stabilisateurs de mousse, des agents propulseurs, des colorants, des agents acidifiants ou alcalinisants ou d'autres adjuvants selon l'usage envisagé.

Les compositions dermatologiques contiennent en outre une substance active pour le traitement des affections dermatologiques.

Les procédés de lavage et/ou de conditionnement des cheveux ou de la peau consistent à appliquer sur ceux-ci une composition telle que définie ci-dessus, cette application étant suivie d'un rinçage.

Les compositions conformes à l'invention sont également utilisables comme gels douche pour le lavage des cheveux et de la peau, auquel cas ils sont appliqués sur la peau et les cheveux humides et sont rincés après application.

Les exemples qui suivent sont destinés à illustrer l'invention sans pour autant présenter un caractère limitatif.

### EXEMPLE 1

On prépare la composition suivante :

| | |
|---|---|
| - Lauryléthersulfate de sodium | 10,0 g |
| - Laurylsulfate d'ammonium | 8,0 g MA |
| - Chlorure de sodium | 3,25 g |
| - Composé de formule (I), dans laquelle : R₁=C₁₆H₃₃, R₂=C₁₄H₂₉ X=O, Y=CH₂ préparé par réaction de 3 moles d'alcool sur 1 mole d'époxyde, utilisé brut | 2,5 g |
| - Monoisopropanolamide d'acide de coprah | 2,0 g |
| - Isooctahexacontane (composé de formule (I) dans laquelle n=16)) | 0,5 g |
| - Isohexadécane (composé de formule (I) dans laquelle n=3)) | 0,8 g |
| - Conservateurs, parfums | |
| - Eau | qsp 100,0 g |
| - pH ajusté à 6,3 avec hydroxyde de sodium | |

Cette composition est utilisée comme shampooing pour le lavage des cheveux.

### EXEMPLE 2

On prépare la composition suivante :

| | |
|---|---|
| - Lauryléthersulfate de sodium | 1,5 g MA |
| - Laurylbétaïne vendue par la Société HENKEL sous la dénomination commerciale DEHYTON AB 30 en solution aqueuse à 32% de MA | 2,5 g MA |
| - Isohexadécane (Perméthyl 101A de PRESPERSE INC.) (Composé de formule (I) dans laquelle n=3)) | 2,5 g |
| - Composé de formule (I), dans laquelle : R₁=C₁₆H₃₃, R₂=C₁₄H₂₉ X=O, Y=CH₂ préparé par réaction de 3 moles d'alcool sur 1 mole d'époxyde, utilisé brut | 2,5 g |
| - Monoéthanolamide d'acide alkyl(C₁₃-C₁₅) éthercarboxylique oxyéthyléné à 2 moles d'oxyde d'éthylène, vendu sous la dénomination AMINOL A 15 par la Société CHEM Y | 2,0 g |
| - Conservateurs, parfums, colorants | |
| - Eau | qsp 100,0 g |
| - pH ajusté à 6,5 avec hydroxyde de sodium | |

Cette composition est utilisée comme shampooing pour le lavage des cheveux.

### EXEMPLE 3

On prépare la composition suivante :

| | |
|---|---|
| - Laurylsulfate d'ammonium | 8,0 g |
| - Laurylsarcosinate de sodium | 5,0 g |
| - Composé de formule (I), dans laquelle : R₁=C₁₂H₂₅ X=O, Y=CH₂ R₂=C₁₂H₂₅/C₁₄H₂₉(50/50 en mole) préparé par réaction de 3 moles d'alcool sur 1 mole d'époxyde, puis distillation de l'alcool excédentaire | 3,0 g |
| - Heptaméthylnonane (composé de formule (I) dans laquelle n=3)) (ARLAMOL HD d'ICI) | 1,0 g |
| - HCl qs pH 6,5 | |
| - Conservateurs, parfums qs | |
| - Eau | qsp 100,0 g |

Cette composition est utilisée comme shampooing pour le lavage des cheveux.

### EXEMPLE 4

On prépare la composition de gel douche suivante :

| | |
|---|---|
| - Alkyl(C₈-C₁₀)polyglucoside en solution aqueuse à 60% de MA, vendu sous la dénomination TRITON CG 110 par la Société SEPPIC | 45,0 g MA |
| - Composé de formule (I), dans laquelle : R₁=C₁₆H₃₃ R₂=C₁₄H₂₉ X=O, Y=CH₂ préparé par réaction de 3 moles d'alcool par mole d'époxyde, utilisé brut | 0,5 g |
| - Perhydrosqualène vendu sous la dénomination COSBIOL par la Société LASERSON SABETAY | 0,1 g |
| - Chlorure de cétyltriméthylammonium en solution aqueuse à 25% de MA, vendu sous la dénomination DEHYQUART A par la Société HENKEL | 1,0 g MA |
| - Parfum, conservateur, colorant qs | |
| - Eau | qsp 100,0 g |
| - pH ajusté à 6 par de la triéthanolamine | |

### EXEMPLE 5

On prépare la composition de gel douche suivante :

| | |
|---|---|
| - Alkyl(C₁₀-C₁₂-C₁₄)polyglucoside (nombre moyen de motif glucoside=1,4) en solution aqueuse à 55% de MA, vendu sous la dénomination ORAMIX WS 10 par la Société SEPPIC | 45,0 g MA |
| - Composé de formule (I), dans laquelle : R₁=C₁₆H₃₃ R₂=C₁₄H₂₉ X=O, Y=CH₂ préparé par réaction de 3 moles d'alcool par mole d'époxyde, utilisé brut | 0,5 g |
| - Huile de Colza (triglycérides d'acides oléïque, linoléique, linolénique 50/20/10) | 10,0 g |
| - Chlorure de cétyltriméthylammonium en solution aqueuse à 25% de MA, vendu sous la dénomination DEHYQUART A par la Société HENKEL | 1,0 g MA |
| - Colorant, conservateur, parfum qs | |
| - Eau | qsp 100,0 g |
| - pH ajusté à 6 par la triéthanolamine | |

### EXEMPLE 6

On prépare le shampooing suivant :

| | |
|---|---|
| - Alkyl(C₁₂-C₁₄)éthersulfate de sodium à 2 moles d'oxyde d'éthylène, vendu sous la dénomination EMPICOL ESB/3 FL par la Société HENKEL en solution aqueuse à 28% de MA | 17,0 g MA |
| - Cocamidoéthyl (N-hydroxyéthyl, N-carboxyméthyl) glycinate de sodium | 8,0 g |
| - Hexadécyloxy-3' hydroxy-2' propyloxy-1 hexadécane, composé de formule (I), dans laquelle : R₁=R₂=C₁₆H₃₃ X=Y=oxygène | 10,0 g |
| - Huile de Colza | 0,1 g |
| - Colorant, conservateur, parfum qs | |
| - Eau | qsp 100,0 g |

## Revendications

1. Composition de lavage des matières kératiniques, en particulier des cheveux et/ou de la peau, caractérisée par le fait qu'elle comprend dans un milieu aqueux, au moins une huile hydrocarbonée, au moins un agent tensio-actif possédant des propriétés détergentes et au moins un alcool ayant 27 à 44 atomes de carbone et comportant un ou deux groupements éther et/ou thioéther ou sulfoxyde, répondant à la formule (I) :
R₁ - X -[C₂H₃(OH)]- CH₂ - Y - R₂ (I)
dans laquelle R₁ et R₂, identiques ou différents, désignent des groupements alkyle linéaires en C₁₂ à C₂₀;
X désigne un atome d'oxygène, un atome de soufre ou un groupement sulfoxyde;
Y désigne un atome d'oxygène, un atome de soufre, un groupement sulfoxyde ou un groupement méthylène;
dans le cas où Y désigne un groupement méthylène, la somme des nombres d'atomes de carbone de R₁ à R₂ varie de 24 à 40, et de préférence de 26 à 36 inclus, et lorsque Y ne désigne pas un groupement méthylène, la somme des atomes de carbone de R₁ et R₂ varie de 24 à 40 inclus, et de préférence de 28 à 36 inclus; lorsque X ou Y désigne sulfoxyde, Y ou X ne désigne pas soufre.

2. Composition selon la revendication 1, caractérisée par le fait que l'alcool ayant 27 à 44 atomes de carbone répond à la formule (I) où X désigne oxygène, Y désigne méthylène et R₁ et R₂ désignent des radicaux ayant 12 à 18 atomes de carbone.

3. Composition selon la revendication 1, caractérisée par le fait que le composé de formule (I) est un composé répondant à la formule :
R₁ - X₁ -[C₂H₃(OH)]- CH₂ - Y₁ - R₂ (IV)
dans laquelle R₁ et R₂ ont la même signification que dans la revendication 1; X₁ désigne un atome d'oxygène ou un groupement sulfoxyde; Y₁ désigne un atome d'oxygène, un groupement méthylène ou sulfoxyde, au moins l'un des symboles X₁ ou Y₁ représente un groupement sulfoxyde.

4. Composition selon l'une quelconque des revendications 1 à 3, caractérisée par le fait que l'huile hydrocarbonée est choisie parmi les huiles de synthèse, les huiles minérales, végétales ou animales, les alcools gras insaturés, les esters d'acides gras et de mono- ou polyalcools en C₂-C₄.

5. Composition selon la revendication 4, caractérisée par le fait que les huiles de synthèse sont choisies parmi les isoparaffines répondant à la formule : 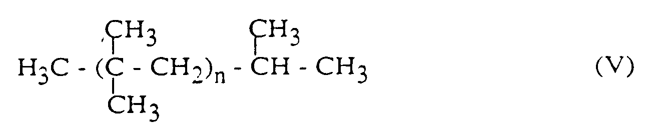 dans laquelle n varie de 2 à 16.

6. Composition selon l'une quelconque des revendications 1 à 5, caractérisée par le fait que l'huile de synthèse est constituée d'un mélange d'isoparaffines de formule (V) : 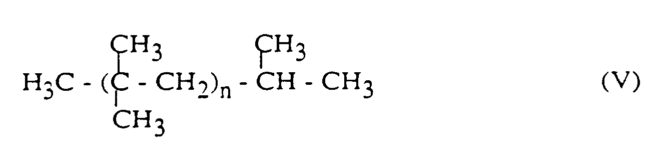 dans laquelle n varie de 2 à 16, et d'isoparaffines répondant à la formule : 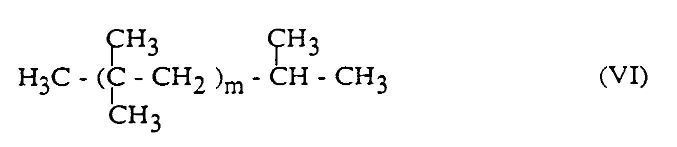 dans laquelle m est supérieur ou égal à 18.

7. Composition selon la revendication 5 ou 6, caractérisée par le fait que les isoparaffines de formules (V) et (VI) sont choisies parmi les huiles de formule (V), dans laquelle n est égal à 2, 3, 4 ou 16, et que m est égal à 38 pour l'isoparaffine de formule (VI).

8. Composition selon l'une quelconque des revendications 1 à 7, caractérisée par le fait que les huiles hydrocarbonées de synthèse sont choisies parmi les triglycérides d'acides gras.

9. Composition selon l'une quelconque des revendications 1 à 4, caractérisée par le fait que l'huile minérale est l'huile de vaseline.

10. Composition selon l'une quelconque des revendications 1 à 4, caractérisée par le fait que les huiles animales sont choisies parmi le squalane, l'huile de baleine, de phoque, de menhaden, de foie de flétan, de foie de morue, de thon, de suif, de boeuf, de cheval, de mouton, de vison, de loutre.

11. Composition selon l'une quelconque des revendications 1 à 4, caractérisée par le fait que l'huile végétale est choisie parmi les huiles d'amande, d'arachide, de germe de blé, de lin, de noyaux d'abricots, de noix, de palme, de pistache, de sésame, d'oeillette, de pin, de ricin, de soja, d'avocat, de carthame, de coco, de noisette, d'olive, de pépins de raisins, de tournesol, de colza, de cade, de germe de maïs, de noyaux de pêches, de café, de jojoba, etc...

12. Composition selon l'une quelconque des revendications 1 à 4 et 11, caractérisée par le fait que l'huile végétale est naturellement ou chimiquement saturée.

13. Composition selon l'une quelconque des revendications 1 à 12, caractérisée par le fait que les agents tensio-actifs détergents sont choisis parmi les agents tensio-actifs anioniques, amphotères, zwittérioniques, non-ioniques ou leurs mélanges.

14. Composition selon la revendication 13, caractérisée par le fait que les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 12 à 20 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 20 atomes de carbone; les acides ethers carboxyliques polyoxyalkylénés.

15. Composition selon la revendication 13, caractérisée par le fait que les agents tensio-actifs non-ioniques sont choisis parmi les alcools ou les alkylphénols ou les acides gras polyéthoxylés, polyoxypropylénés ou polyglycérolés, à chaîne grasse comportant 8 à 18 atomes de carbone, le nombre de groupements oxyde d'éthylène, oxyde de propylène étant compris entre 2 et 50 et le nombre de groupements glycérol étant compris entre 2 et 30; les copolymères oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les oxydes d'amines.

16. Composition selon la revendication 13, caractérisée par le fait que les agents tensio-actifs amphotères ou zwittérioniques sont choisis parmi les dérivés d'amines secondaires ou tertiaires aliphatiques, dans lesquels le radical aliphatique est une chaîne linéaire ou ramifiée comportant de 8 à 18 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

17. Composition selon l'une quelconque des revendications 1 à 16, caractérisée par le fait que l'huile hydrocarbonée est utilisée dans les compositions conformes à l'invention dans des proportions comprises entre 0,05 et 20% et de préférence entre 0,1 et 10% en poids par rapport au poids total de la composition.

18. Composition selon l'une quelconque des revendications 1 à 17, caractérisée par le fait que les alcools à groupements éther et/ou thioéher ou sulfoxyde de formule (I) sont utilisés dans des proportions suffisantes pour assurer la mise en suspension des huiles hydrocarbonées.

19. Composition selon la revendication 18, caractérisée par le fait que les alcools à groupements éther et/ou thioéther sulfoxyde de formule (I) sont présents dans des proportions comprises entre 0,1 et 20% en poids par rapport au poids total de la composition, et en particulier entre 0,5 et 10%.

20. Composition selon l'une quelconque des revendications 1 à 19 caractérisée par le fait que les agents tensio-actifs sont présents dans une proportion suffisante pour conférer un caractère détergent à la composition.

21. Composition selon la revendication 20, caractérisée par le fait que les agents tensio-actifs sont présents dans des proportions comprises entre 5 et 50% en poids par rapport au poids total de la composition et en particulier entre 8 et 35% en poids.

22. Composition selon l'une quelconque des revendications 1 à 21, caractérisée par le fait que le pH est compris entre 3 et 9 et en particulier entre 3 et 8.

23. Composition selon l'une quelconque des revendications 1 à 22, caractérisée par le fait que le milieu aqueux est constitué par de l'eau ou par un mélange d'eau et d'un solvant cosmétiquement acceptable choisi parmi les alcools inférieurs, les alkylèneglycols et les éthers de glycol, l'eau étant présente dans des proportions supérieures à 20%.

24. Composition selon l'une quelconque des revendications 1 à 23, caractérisée par le fait que la composition contient en plus des agents régulateurs de viscosité choisis parmi les électrolytes, les hydrotropes ou des agents épaississants présents en des proportions pouvant aller jusqu'à 15% en poids par rapport au poids total de la composition.

25. Composition selon l'une quelconque des revendications 1 à 24, caractérisée par le fait qu'elle contient en plus un ou plusieurs adjuvants choisis parmi les tensio-actifs cationiques, les polymères anioniques ou non-ioniques ou cationiques ou amphotères, des protéines éventuellement quaternisées ou une huile, cire, gomme ou résine de silicone.

26. Composition selon l'une quelconque des revendications 1 à 25, caractérisée par le fait qu'elle contient différents adjuvants cosmétiquement acceptables choisis parmi les parfums, les conservateurs, les séquestrants, les synergistes de mousses, les stabilisateurs de mousses, les agents acidifiants ou alcalinisants.

27. Utilisation comme shampooing de la composition telle que définie dans l'une quelconque des revendications 1 à 26.

28. Utilisation comme gel douche de la composition telle que définie dans l'une quelconque des revendications 1 à 26.

29. Procédé de lavage et de conditionnement des matières kératiniques, caractérisé par le fait que l'on applique sur ces matières au moins une composition telle que définie dans l'une quelconque des revendications 1 à 26, et qu'après un temps de pose on rince à l'eau les matières traitées.

30. Utilisation d'un alcool ayant 27 à 44 atomes de carbone et comportant un ou deux groupements éther et/ou thioéther ou sulfoxyde, répondant à la formule (I) :
R₁ - X -[C₂H₃(OH)]- CH₂ - Y - R₂ (I)
dans laquelle R₁ et R₂, identiques ou différents, désignent des groupements alkyle linéaires en C₁₂ à C₂₀;
X désigne un atome d'oxygène, un atome de soufre ou un groupement sulfoxyde;
Y désigne un atome d'oxygène, un atome de soufre, un groupement sulfoxyde ou un groupement méthylène;
dans le cas où Y désigne un groupement méthylène, la somme des nombres d'atomes de carbone de R₁ à R₂ varie de 24 à 40, et de préférence de 26 à 36 inclus, et lorsque Y ne désigne pas un groupement méthylène, la somme des atomes de carbone de R₁ et R₂ varie de 24 à 40 inclus, et de préférence de 28 à 36 inclus; lorsque X ou Y désigne sulfoxyde, Y ou X ne désigne pas soufre;
comme agents de mise en suspension d'huiles hydrocarbonées dans un milieu aqueux contenant des agents tensio-actifs détergents.

## Claims

1. Composition for washing keratinous materials, in particular the hair and/or the skin, characterised in that it comprises, in an aqueous medium, at least one hydrocarbon oil, at least one surface-active agent possessing detergent properties and at least one alcohol having 27 to 44 carbon atoms and comprising one or two ether and/or thioether or sulphoxide group(s) of the formula (I):
R₁-X-[C₂H₃(OH)]-CH₂-Y-R₂ (I)
in which R₁ and R₂, which are identical or different, denote linear C₁₂ to C₂₀ alkyl groups;
X denotes an oxygen atom, a sulphur atom or a sulphoxide group;
Y denotes an oxygen or sulphur atom, a sulphoxide group or a methylene group;
in the case where Y denotes a methylene group, the sum of the number of carbon atoms in R₁ to R₂ ranges from 24 to 40 and preferably from 26 to 36 inclusive, and when Y does not denote a methylene group, the sum of the R₁ and R₂ carbon atoms ranges from 24 to 40 inclusive and preferably from 28 to 36 inclusive; when X or Y denotes sulphoxide, Y or X does not denote sulphur.

2. Composition according to Claim 1, characterised in that the alcohol having 27 to 44 carbon atoms is of the formula (I) where X denotes oxygen, Y denotes methylene and R₁ and R₂ denote radicals having 12 to 18 carbon atoms.

3. Composition according to Claim 1, characterised in that the compound of formula (I) is a compound of the formula:
R₁ - X₁ -[C₂H₃(OH)]-CH₂ - Y₁ - R₂ (IV)
in which R₁ and R₂ have the same meaning as in Claim 1; X₁ denotes an oxygen atom or a sulphoxide group; Y₁ denotes an oxygen atom, a methylene or sulphoxide group, at least one of the symbols X₁ or Y₁ represents a sulphoxide group.

4. Composition according to any one of Claims 1 to 3, characterised in that the hydrocarbon oil is chosen from synthetic oils, mineral, vegetable or animal oils, unsaturated fatty alcohols, and esters of fatty acids and C₂-C₄ mono- or polyalcohols.

5. Composition according to Claim 4, characterised in that the synthetic oils are chosen from the isoparaffins of the formula: 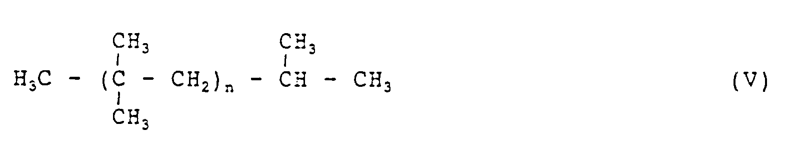 in which n ranges from 2 to 16.

6. Composition according to any one of Claims 1 to 5, characterised in that the synthetic oil is composed of a mixture of isoparaffins of formula (V): 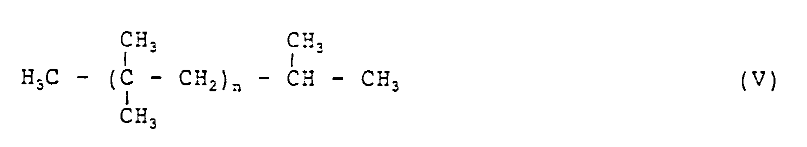 in which n ranges from 2 to 16, and of isoparaffins of the formula: 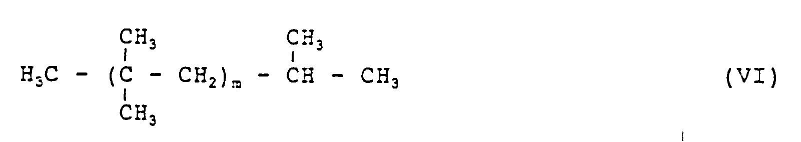 in which m is not less than 18.

7. Composition according to Claim 5 or 6, characterised in that the isoparaffins of formulae (V) and (VI) are chosen from the oils of formula (V), in which n is equal to 2, 3, 4 or 16, and in that m is equal to 38 for the isoparaffin of formula (VI).

8. Composition according to any one of Claims 1 to 7, characterised in that the synthetic hydrocarbon oils are chosen from fatty acid triglycerides.

9. Composition according to any one of Claims 1 to 4, characterised in that the mineral oil is liquid paraffin.

10. Composition according to any one of Claims 1 to 4, characterised in that the animal oils are chosen from squalene and from whale, seal, menhaden, halibut liver, cod liver, tuna fish, tallow, beef, caballine, mutton, vison or otter oil.

11. Composition according to any one of Claims 1 to 4, characterised in that the vegetable oil is chosen from almond, peanut, wheat-germ, linseed, apricot stone, nut, palm, pistachio, sesame, poppy seed, pine, castor, soya bean, avocado, safflower, coconut, hazelnut, olive, grape seed, sunflower, colza, cade, maize-germ, peach stone, coffee, jojoba oils and the like.

12. Composition according to any one of Claims 1 to 4 and 11, characterised in that the vegetable oil is naturally or chemically saturated.

13. Composition according to any one of Claims 1 to 12, characterised in that the detergent surface-active agents are chosen from anionic, amphoteric, zwitterionic, non-ionic surface-active agents or their mixtures.

14. Composition according to Claim 13, characterised in that the anionic surface-active agents are chosen from the alkali metal salts, the magnesium salts, the ammonium salts, the amine salts or the aminoalcohol salts of the following compounds: alkyl sulphates, alkyl ether sulphates, alkyl amidoether sulphates, alkyl aryl polyether sulphates, monoglyceride sulphates; alkyl sulphonates, alkyl amide sulphonates, alkyl aryl sulphonates, olefin sulphonates, paraffin sulphonates; alkyl sulphosuccinates, alkyl ether sulphosuccinates, alkyl amide sulphosuccinates; alkyl sulphosuccinamates; alkyl sulphoacetates; alkyl phosphates, alkyl ether phosphates; acyl sarcosinates, acyl isethionates, N-acyltaurates; the alkyl or acyl radical of these various compounds consisting of a carbon chain containing 12 to 20 carbon atoms; fatty acid salts of oleic, ricinoleic, palmitic and stearic acids; copra oil or hydrogenated copra oil acids; acyl lactylates whose acyl radical contains 8 to 20 carbon atoms; polyoxyalkylenated carboxylic ether acids.

15. Composition according to Claim 13, characterised in that the non-ionic surface-active agents are chosen from polyethoxylated, polyoxypropylenated or polyglycerolated alcohols or alkylphenols or fatty acids, with a fatty chain containing 8 to 18 carbon atoms, the number of ethylene oxide or propylene oxide groups being between 2 and 50 and the number of glycerol groups being between 2 and 30; ethylene and propylene oxide copolymers; condensates of ethylene and propylene oxides with fatty alcohols; polyethoxylated fatty amides; polyglycerolated fatty amides, polyethoxylated fatty amines; oxyethylenated sorbitan fatty acid esters; sucrose or polyethylene glycol fatty acid esters; alkyl polyglycosides; amine oxides.

16. Composition according to Claim 13, characterised in that the amphoteric or zwitterionic surface-active agents are chosen from the aliphatic secondary or tertiary amine derivatives in which the aliphatic radical is a linear or branched chain containing 8 to 18 carbon atoms and which contains at least one anionic water-solubilising carboxylate, sulphonate, sulphate, phosphate or phosphonate group; (C₈-C₂₀ alkyl)betaines, sulphobetaines, (C₈-C₂₀ alkyl)amido(C₁-C₆ alkyl)betaines or (C₈-C₂₀ alkyl)amido(C₁-C₆ alkyl)sulphobetaines.

17. Composition according to any one of Claims 1 to 16, characterised in that the hydrocarbon oil is used in the compositions conforming to the invention in proportions of between 0.05 and 20% and preferably between 0.1 and 10% by weight relative to the total weight of the composition.

18. Composition according to any one of Claims 1 to 17, characterised in that the alcohols containing ether and/or thioether or sulphoxide groups of formula (I) are used in proportions sufficient to ensure the suspension of the hydrocarbon oils.

19. Composition according to Claim 18, characterised in that the alcohols containing ether and/or thioether or sulphoxide groups of formula (I) are present in proportions of between 0.1 and 20% by weight relative to the total weight of the composition and in particular between 0.5 and 10%.

20. Composition according to any one of Claims 1 to 19, characterised in that the surface-active agents are present in a proportion sufficient to confer a detergent character on the composition.

21. Composition according to Claim 20, characterised in that the surface-active agents are present in proportions of between 5 and 50% by weight relative to the total weight of the composition and in particular between 8 and 35% by weight.

22. Composition according to any one of Claims 1 to 21, characterised in that the pH is between 3 and 9 and in particular between 3 and 8.

23. Composition according to any one of Claims 1 to 22, characterised in that the aqueous medium is composed of water or a mixture of water and a cosmetically acceptable solvent chosen from lower alcohols, alkylene glycols and glycol ethers, the water being present in proportions above 20%.

24. Composition according to any one of Claims 1 to 23, characterised in that the composition in addition contains viscosity regulating agents chosen from electrolytes, hydrotropic agents or thickening agents present in proportions which may range up to 15% by weight relative to the total weight of the composition.

25. Composition according to any one of Claims 1 to 24, characterised in that it in addition contains one or more adjuvants chosen from cationic surface-active agents, anionic or non-ionic or cationic or amphoteric polymers, optionally quaternised proteins or a silicone oil, wax, gum or resin.

26. Composition according to any one of Claims 1 to 25, characterised in that it contains various cosmetically acceptable adjuvants chosen from perfumes, preservatives, sequestrants, foam synergists, foam stabilisers and acidifying or alkalinising agents.

27. Use as shampoo of the composition as defined in any one of Claims 1 to 26.

28. Use as shower gel of the composition as defined in any one of Claims 1 to 26.

29. Method for washing and conditioning keratinous materials, characterised in that at least one composition as defined in any one of Claims 1 to 26 is applied to these materials, and in that after a period of exposure, the treated materials are rinsed with water.

30. Use of an alcohol having 27 to 44 carbon atoms and containing one or two ether and/or thioether or sulphoxide groups, of the formula (I):
R₁ - X -[C₂H₃(OH)]-CH₂ - Y - R₂ (I)
in which R₁ and R₂, which are identical or different, denote linear C₁₂ to C₂₀ alkyl groups;
X denotes an oxygen atom, a sulphur atom or a sulphoxide group;
Y denotes an oxygen atom, a sulphur atom, a sulphoxide group or a methylene group;
in the case where Y denotes a methylene group, the sum of the number of carbon atoms in R₁ to R₂ ranges from 24 to 40 and preferably from 26 to 36 inclusive, and when Y does not denote a methylene group, the sum of the R₁ and R₂ carbon atoms ranges from 24 to 40 inclusive and preferably from 28 to 36 inclusive; when X or Y denotes sulphoxide, Y or X does not denote sulphur;
as agents for suspending hydrocarbon oils in an aqueous medium containing detergent surface-active agents.

## Patentansprüche

1. Zusammensetzung zum Waschen keratinischer Materien, insbesondere der Haare und/oder der Haut,
dadurch **gekennzeichnet**, daß
sie in einem wässrigen Medium mindestens ein Kohlenwasserstofföl, mindestens ein oberflächenaktives Mittel, das Detergens- bzw. Reinigungsmitteleigenschaften besitzt, sowie mindestens einen Alkohol enthält, der 27 bis 44 Kohlenstoffatome, eine oder zwei Ether- und/oder Thioether- oder Sulfoxidgruppen und die Formel (I) aufweist:
R₁ - X-(C₂H₃(OH))-CH₂-Y-R₂ (I)
worin R₁ und R₂, unabhängig voneinander, lineare C₁₂₋₂₀-Alkylgruppen,
X ein Sauerstoffatom, ein Schwefelatom oder eine Sulfoxidgruppe und
Y ein Sauerstoff-, Schwefelatom, eine Sulfoxid- oder Methylengruppe darstellen,
und wobei im Fall, daß Y eine Methylengruppe darstellt, die Summe der Zahl der in den Gruppen R₁ und R₂ vorhandenen Kohlenstoffatome einen Wert von 24 bis 40 und vorzugsweise von 26 bis 36 aufweist,
und wenn Y keine Methylengruppe darstellt, die Summe der Kohlenstoffatome von R₁ und R₂ einen Wert von 24 bis 40 und vorzugsweise von 28 bis 36 aufweist,
und wenn X oder Y eine Sulfoxidgruppe darstellen, Y oder X kein Schwefelatom bedeuten.

2. Zusammensetzung gemäß Anspruch 1,
dadurch **gekennzeichnet**, daß
der Alkohol mit 27 bis 44 Kohlenstoffatomen die Formel (I) aufweist, worin X Sauerstoff, Y eine Methylengruppe und R₁ und R₂ Reste mit 12 bis 18 Kohlenstoffatomen darstellen.

3. Zusammensetzung gemäß Anspruch 1,
dadurch **gekennzeichnet**, daß
die Verbindung der Formel (I) eine Verbindung der Formel ist:
R₁ - X₁ -[C₂H₃(OH)]- CH₂ - Y₁ - R₂ (IV)
worin R₁ und R₂ dieselbe Bedeutung wie in der Formel (I) haben, X₁ ein Sauerstoffatom oder eine Sulfoxidgruppe, Y₁ ein Sauerstoffatom, eine Methylen- oder Sulfoxidgruppe bedeuten, und mindestens eines der Symbole X₁ oder Y₁ eine Sulfoxidgruppe darstellen.

4. Zusammensetzung gemäß einem jeden der Ansprüche 1 bis 3,
dadurch **gekennzeichnet**, daß
das Kohlenwasserstofföl aus Syntheseölen, mineralischen, pflanzlichen oder tierischen Ölen, ungesättigten Fettalkoholen, Estern von Fettsäuren und von C₂₋₄-Mono- oder -polyalkoholen ausgewählt ist.

5. Zusammensetzung gemäß Anspruch 4,
dadurch **gekennzeichnet**, daß
die Syntheseöle aus Isoparaffinen der Formel ausgewählt sind: 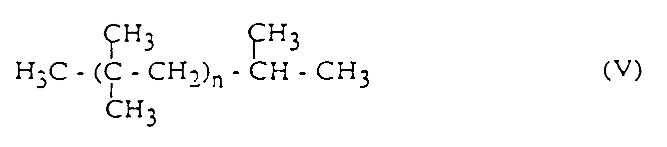 worin n 2 bis 16 beträgt.

6. Zusammensetzung gemäß einem jeden der Ansprüche 1 bis 5,
dadurch **gekennzeichnet**, daß
das Syntheseöl aus einer Mischung von Isoparaffinen der Formel (V): 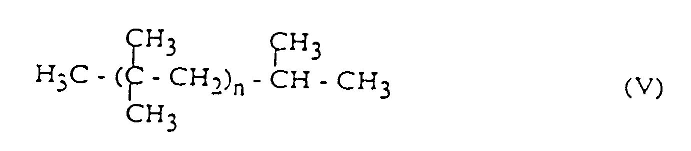 worin n 2 bis 16 beträgt, und von Isoparaffinen der Formel zusammengesetzt ist: 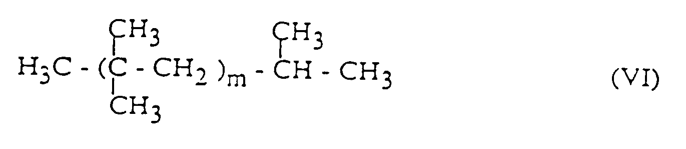 worin m 18 oder mehr beträgt.

7. Zusammensetzung gemäß Anspurch 5 oder 6,
dadurch **gekennzeichnet**, daß
die Isoparaffine der Formeln (V) und (VI) aus Ölen der Formel (V), worin n 2, 3, 4 oder 16 ist, und aus einem Öl der Formel (VI) augewählt sind, worin m für das Isoparaffin der Formel (VI) gleich 38 ist.

8. Zusammensetzung gemäß einem jeden der Ansprüche 1 bis 7,
dadurch **gekennzeichnet**, daß
die Kohlenwasserstoffsyntheseöle aus Triglyceriden von Fettsäuren ausgewählt sind.

9. Zusammensetzung gemäß einem jeden der Ansprüche 1 bis 4,
dadurch **gekennzeichnet**, daß
das Mineralöl Vaselineöl ist.

10. Zusammensetzung gemäß einem jeden der Ansprüche 1 bis 4,
dadurch **gekennzeichnet**, daß
die tierischen Öle aus Squalan, dem Öl von Walfisch, Robbe, Menhaden, Flunderleber, Kabeljauleber, Thunfisch, Talg, Rind, Pferd, Schaf, Nerz und Fischotter ausgewählt sind.

11. Zusammensetzung gemäß einem jeden der Ansprüche 1 bis 4,
dadurch **gekennzeichnet**, daß
das pflanzliche Öl aus Ölen von Mandel, Erdnuß, Weizenkeimen, Leinsamen, Aprikosenkernen, Nuß, Palme, Pistazie, Sesam, Ölbaum, Pinie, Rizinus, Soja, Avocado, Karthame, Kokos, Nußbaum, Ölive, Rosinenkernen, Sonnenblumen, Raps, Kade, Maiskeimen, Pfirsichkernen, Kaffee, Jojoba usw. ausgewählt ist.

12. Zusammensetzung gemäß einem jeden der Ansprüche 1 bis 4 und 11,
dadurch **gekennzeichnet**, daß
das pflanzliche Öl ein natürlich oder chemisch gesättigtes ist.

13. Zusammensetzung gemäß einem jeden der Ansprüche 1 bis 12,
dadurch **gekennzeichnet**, daß
die oberflächenaktiven Detergentien aus oberflächenaktiven anionischen, amphoteren, zwitterionischen, nicht-ionischen Mitteln oder aus deren Mischungen ausgewählt sind.

14. Zusammensetzung gemäß Anspruch 13,
dadurch **gekennzeichnet**, daß
die oberflächenaktiven anionischen Mittel aus Alkali-, Magnesium-, Ammonium-, Amin- oder Aminoalkoholsalzen der folgenden Verbindungen: von Alkylsulfaten, Alkylethersulfaten, Alkylamidoethersulfaten, Alkylarylpolyethersulfaten, Monoglyceridsulfaten, Alkylsulfonaten, Alkylamidsulfonaten, Alkylarylsulfonaten, Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetaten, Alkylphosphaten, Alkyletherphosphaten, Acylsarcosinaten, Acylisethionaten und N-Acyltauraten, wobei der Alkyl- oder Acylrest dieser verschiedenen Verbindungen aus einer Kohlenwasserstoffkette mit 12 bis 20 Kohlenstoffatomen zusammengesetzt ist, aus Fettsäuresalzen der Öl-, Rizinolein-, Palmitin- und Stearinsäure, aus Säuren vom Öl von Koprah oder von hydriertem Koprah, aus Acyllactylaten, deren Acylrest 8 bis 20 Kohlenstofftome enthält, sowie aus polyoxalkylierten Ethercarboxylsäuren ausgewählt sind.

15. Zusammensetzung gemäß Anspruch 13,
dadurch **gekennzeichnet**, daß
die oberflächenaktiven nicht-ionischen Mittel aus polyethoxylierten, polyoxpropylierten oder polyglycerierten Alkoholen oder Alkylphenolen oder Fettsäuren mit einer Fettkette von 8 bis 18 Kohlenstoffatomen, wobei die Anzahl der Ethylenoxid- oder Propylenoxidgruppen 2 bis 50 und der Glyceringruppen 2 bis 30 beträgt, aus Copolymeren von Ethylen- und Propylenoxid, aus Kondensaten von Ethylen- und Propylenoxid mit Fettalkoholen, aus polyethoxylierten Fettamiden, aus polyglycerierten Fettamiden, aus polyethoxylierten Fettaminen, aus oxethylierten Fettsäureestern des Sorbitans, aus Fettsäureestern von Succrose oder des Polyethylenglycols, aus Alkylpolyglycosiden und aus Aminoxiden ausgewählt sind.

16. Zusammensetzung gemäß Anspruch 13,
dadurch **gekennzeichnet**, daß
die oberflächenaktiven amphoteren oder zwitterionischen Mittel aus aliphatischen sekundären oder tertiären Aminderivaten, in denen der aliphatische Rest eine lineare oder verzweigte Kette mit 8 bis 18 Kohlenstoffatomen ist, und mindestens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder aus C₈₋₂₀-Alkylamido-C₁₋₆-alkylsulfobetainen ausgewählt sind.

17. Zusammensetzung gemäß einem jeden der Ansprüche 1 bis 16,
dadurch **gekennzeichnet**, daß
das Kohlenwasserstofföl in den erfindungsgemäßen Zusammensetzungen in Mengenverhältnissen von 0,05 bis 20 Gew.%, vorzugsweise von 0,1 bis 10 Gew.%, bezogen auf das Gesamtgewicht der Zusammensetzung, verwendet wird.

18. Zusammensetzung gemäß einem jeden der Ansprüche 1 bis 17,
dadurch **gekennzeichnet**, daß
die Alkohole mit Ether- und/oder Thioether- oder Sulfoxidgruppe der Formel (I) in Mengenverhältnissen verwendet werden, die ausreichen, um das Suspendieren der Kohlenwasserstofföle zu gewährleisten.

19. Zusammensetzung gemäß Anspruch 18,
dadurch **gekennzeichnet**, daß
die Alkohole mit Ether- und/oder Thioether- oder Sulfoxidgruppe der Formel (I) in Mengenverhältnissen von 0,1 bis 20 Gew.%, insbesondere von 0,5 bis 10 Gew.%, bezogen auf das Gesamtgewicht der Zusammensetzung, vorhanden sind.

20. Zusammensetzung gemäß einem jeden der Ansprüche 1 bis 19,
dadurch **gekennzeichnet**, daß
die oberflächenaktiven Mittel in einem Mengenverhältnis vorliegen, das ausreicht, um der Zusammensetzung Detergens- bzw. Reinigungsmitteleigenschaften zu verleihen.

21. Zusammensetzung gemäß Anspruch 20,
dadurch **gekennzeichnet**, daß
die oberflächenkativen Mittel in Mengenverhältnissen von 5 bis 50 Gew.%, insbesondere von 8 bis 35 Gew.%, bezogen auf das Gesamtgewicht der Zusammensetzung, vorhanden sind.

22. Zusammensetzung gemäß einem jeden der Ansprüche 1 bis 21,
dadurch **gekennzeichnet**, daß
der pH 3 bis 9 und insbesondere 3 bis 8 beträgt.

23. Zusammensetzung gemäß einem jeden der Ansprüche 1 bis 22,
dadurch **gekennzeichnet**, daß
das wässrige Medium aus Wasser oder einer Mischung aus Wasser und einem kosmetisch geeigneten Lösungsmittel aus Niedrigalkoholen, Alkylenglycolen und Glycolethern zusammengesetzt ist, wobei das Wasser in Mengenverhältnissen von mehr als 20% vorhanden ist.

24. Zusammensetzung gemäß einem jeden der Ansprüche 1 bis 23,
dadurch **gekennzeichnet**, daß
die Zusammensetzung zusätzlich Viskositätsregulierungsmittel aus Elektrolyten, Hydrotropen oder Verdickungsmitteln enthält, die in Mengenverhältnissen bis zu 15 Gew.% vorhanden sind, bezogen auf das Gesamtgewicht der Zusammensetzung.

25. Zusammensetzung gemäß einem jeden der Ansprüche 1 bis 24,
dadurch **gekennzeichnet**, daß
sie zusätzlich einen oder mehrere Hilfsstoffe enthält, ausgewählt aus kationischen oberflächenaktiven Mitteln, anionischen oder nicht-ionischen oder kationischen oder amphoteren Polymeren, gegebenenfalls quaternierten Proteinen oder aus einem Öl, Wachs, einem Gummi- oder Harzprodukt von Silikon.

26. Zusammensetzung gemäß einem jeden der Ansprüche 1 bis 25,
dadurch **gekennzeichnet**, daß
sie verschiedene, kosmetisch geeignete Hilfsstoffe enthält, ausgewählt aus Parfüm-Produkten, Konservierungsstoffen, Sequestriermitteln, Schaum-Synergisten, Schaumstabilisatoren, sauer oder alkalisch machenden Mitteln.

27. Verwendung der in einem jeden der Ansprüche 1 bis 26 definierten Zusammensetzung als Shampoo.

28. Verwendung der in einem jeden der Ansprüche 1 bis 26 definierten Zusammensetzung als Duschgel.

29. Verfahren zum Waschen und Konditionieren keratinischer Materien,
dadurch **gekennzeichnet**, daß
man auf diese Materien mindestens eine in jedem der Ansprüche 1 bis 26 definierte Zusammensetzung aufbringt und nach einer Verweilzeit die behandelten Materien mit Wasser spült.

30. Verwendung eines Alkohols mit 27 bis 44 Kohlenstoffatomen und einer oder zwei Ether- und/oder Thioether- oder Sulfoxidgruppen der Formel (I):
R₁ - X-(C₂H₃(OH))-CH₂-Y-R₂ (I)
worin R₁ und R₂, unabhängig voneinander, lineare C₁₂₋₂₀-Alkylgruppen,
X ein Sauerstoffatom, ein Schwefelatom, eine Sulfoxidgruppe und
Y ein Sauerstoff-, Schwefelatom, eine Sulfoxid- oder Methylengruppe darstellen,
und wobei im Fall, daß Y eine Methylengruppe darstellt, die Summe der Zahl der in den Gruppen R₁ und R₂ vorhandenen Kohlenstoffatome einen Wert von 24 bis 40 und vorzugsweise von 26 bis 36 aufweist,
und wenn Y keine Methylengruppe darstellt, die Summe der Kohlenstoffatome von R₁ und R₂ einen Wert von 24 bis 40 und vorzugsweise von 28 bis 36 aufweist,
und wenn X oder Y eine Sulfoxidgruppe darstellen, Y oder X kein Schwefelatom bedeuten,
als Mittel zum Suspendieren von Kohlenwasserstoffölen in einem wässrigen Medium, das oberflächenaktive Detergentien enthält.
